# EUROPEAN PATENT APPLICATION

(11) **EP 1 281 412 A1**
(43) Date of publication of application: **05.02.2003**
(21) Application number: 02380052.7
(22) Date of filing: 06.03.2002
(51) Int. Cl.: A61M 5/50

(54) **Non reusable syringe**

(30) Priority: 07.03.2001 ES 200100536
(71) Applicant: Eurochina Farma S.L., Carlet, 46240 Valencia (ES)
(72) Inventor: Rico Bautista, Leonardo, 46013 Valencia (ES)

(57) **Abstract**

The object of the invention is a disposable safety syringe which is destroyed after use. The base (2) of the cylindrical body has a cone (3) which pierces a disc (6) of the plunger when the plunger is fully pushed into the body. When another attempt is made to suck in liquid, air passes through the box (7) and the hole of the pierced disc (6) of the plunger. This makes it impossible to obtain a vacuum for suckink in liquid into the syringe. Hence, the syringe is only usable one time.

## Description

### OBJECT OF THE INVENTION

The object of invention protected in this Patent consist on a "New disposable syringe", one of the used to inject liquid medicine into weaves or organs.

### ANTECEDENTS

The known structure of the syringes for this use consist of:
A hollow cylindrical body which in one of its closed bases has a tubular form of minor diameter. In this tubular form a hollow, on angle in vertix and very acute needle is joined.
An elastic plunger that fastenly slides inside the cylindric body to suck in and propel alternately the liquid serum.
A rod fastened in one side to the center of one of the plunger faces that transfers the movement with the mechanic requests to traction or compression. It receives these requests by hand in the oposite side which has a plane circular and bigger diameter to make the application of the requests easier.

The syringes used at the beginning had the same structure described above and were made with vitreous materials that permited their disinfection by immersion into boiling water to make possible its indefinite reuse.

Later the syringes were made of mouldable plastic, this made the above described disinfection imposible and conditioned the prescription of only one use for the plastic syringes.

Anyway the use of the syringes by marginal collectives propitiates the infection of illness of blood trånsmition due to the use of the same syringe by many users, in spite of the continued warnings on the contrary by the plublic information campaigns, it does not stop their multiple use.

### DESCRIPTION OF THE INVENTION

The purpose of the invention that constitudes the object of this Patent consist on the overcoming of the inconvenients of the known syringes above described. It has been designed with this prioritary objective.

The most important of this invention is to achieve, by giving auxiliary ways to the known syringes, technically and objectively impossible their reuse, in spite of the users will.

These ways are placed in the hollow of the syringe and in the plunger because both interact in a complementary way. They consist on:
At the bottom or closed base of the hollow body there is a small cone of angle in the vertix very acute, in a coaxial position in reference to the body, thats why the tubular form remains eccentric for the joining of the needle.
The body of the plunger consist on two planes octagonally cut in the simetric longitudinal axis. The disc of the plunger has in its upper face a small hollow box of rectangular section with its longitudinal axis plainly oriented towards one of the planes of plunger body.
The functioning of the syringe is : The first suck in of the injectable liquid is produced normally but when it is propelled through the hypodermic needle the disck of the plungers arrives to the end of the outside cylindric body the cone perforates it. This makes impossible a second suck in because the air comes in the small box through the interstices placed between the plane basis and the inner cylindric surface outside. It goes from the little box to the inner disc throught the perforation produced preventing the rarefying of the atmosphere in the suck in chamber and therefore the penetration of the injectable liquid in the syringe.

### BRIEF DESCRIPTION OF THE DRAWINGS

To complement the description of the invention and make easier the interpretation of the formal, structural and functional characteristics of its object here enclosed you could see the drawing that schematically represent the aspects of a preferent realization of the "New disposable syringe" which is the object of this Patent. In the drawings:

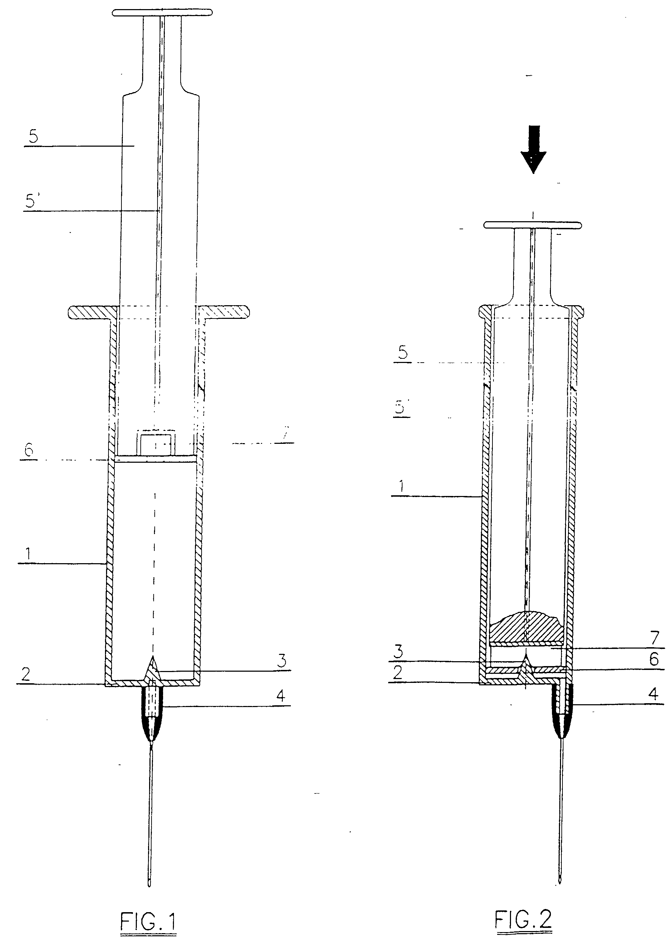

Figure 1 represents the longitudinal section of the outside body of the syringe in the suck in phase. It is in a axial plane that does not cut the plunger, it shows the plunger configuration and the arrangement of its components.
Figure 2 represents the longitudinal section of the syringe in its propeller phase in an axial plane, not cutting the plunger. From the plunger it is only extracted a small zone to show the perforation of the disc by the cone of the base of the outside body at the end of the coming out of the injectable liquid.

### DESCRIPTION OF A PREFERENT REALIZATION

To show clearly the nature and reach of the profitable aplication of the "New disposable syringe" that constitudes the object of the claimed invention here are described its functioning and structure, with reference to the drawings to represent a realization of this object. The functioning of the syringe here explained is to be taken in an informative way and has to be considered in a wide sense and not as limitations of the aplication of the claimed invention.

The end or closed base (2) of the hollow cylindric body (1) has a small cone (3) of angle in the vertix very acute, in a coaxial position in reference to the body (1), by the tubular form (4) for the joining of the hypodermic needle remains eccentric.

The body of the plunger is formed by two planes (5)-(5') octagonally cut in the simetric longitudinal axis, while the disc (6) has in its upper face a small hollow box (7) of rectangular section with its longitudinal axis plainly oriented towards one of the planes (5)-(5') of the plunger body.

The functioning of the syringe is : The first suck in of the injectable liquid is produced normally but when it is propelled through the hypodermic needle the disck (6) of the plungers arrives to the end (2) of the outside cylindric body (1) the cone (3) perforates it. This makes impossible a second suck in because the air comes in the small box (7) through the interstices placed between the plane basis and the inner cylindric surface outside (1). It goes from the little box (7) to the inner disc (6) throught the perforation produced (3) preventing the rarefying of the atmosphere in the suck in chamber and therefore the penetration of the injectable liquid in the syringe.

## Claims

1. This new disposable syringe, is **characterised by** the fact that the end or closed base (2) of the hollow cylindrical body (1) has a small cone (3) with a very acutely angled vertex, which is positioned coaxially to the aforementioned body (1). This means that the nozzle (4) for the insertion of the hypodermic needle is in an eccentric position.

2. This new disposable syringe, in accordance with the first claim, is **characterised by** the fact that the body of the piston is made up of two portions (5)-(5') which cross each other orthogonally on the longitudinal axis of symmetry, while the disc (6) has a small hollow box (7) of rectangular section on its upper face, whose longitudinal axis is positioned on the same plane as one of the two portions (5)-(5') of the piston body.

3. This new disposable syringe, in accordance with the previous claims, is **characterised by** the fact that the first intake of liquid is injected as normal, but as the liquid is propelled through the hypodermic needle, when the disk (6) of the piston reaches the bottom (2) of the outer cylindrical body (1), the cone (3) perforates it. This means that a second intake of liquid is impossible since air enters the box (7) through the interstices between its flat bases and the internal cylindrical surface of the outer body (1), passing the air from the box (7) to the lower face of the disc (6) by means of the perforation caused by the cone (3), thus preventing the creation of a vacuum in the intake chamber and the subsequent intake of injectable liquid in the syringe.
